(19) Europäisches Patentamt
European Patent Office
Office européen des brevets

(11) EP 1 542 740 B1

(12) **EUROPEAN PATENT SPECIFICATION**

(45) Date of publication and mention of the grant of the patent:
**06.05.2009 Bulletin 2009/19**

(21) Application number: **03797902.8**

(22) Date of filing: **10.09.2003**

(51) Int Cl.:
***A61L 31/10*** *(2006.01)* ***A61L 31/16*** *(2006.01)*

(86) International application number:
**PCT/US2003/028643**

(87) International publication number:
**WO 2004/026359 (01.04.2004 Gazette 2004/14)**

# (54) FLUOROPOLYMER COATINGS FOR IMPLANTABLE MEDICAL DEVICES

FLUORPOLYMERBESCHICHTUNGEN FÜR IMPLANTIERBARE MEDIZINISCHE VORRICHTUNGEN

REVETEMENTS EN FLUOROPOLYMERE DESTINES A DES DISPOSITIFS MEDICAUX IMPLANTABLES

(84) Designated Contracting States:
**AT BE BG CH CY CZ DE DK EE ES FI FR GB GR HU IE IT LI LU MC NL PT RO SE SI SK TR**

(30) Priority: **19.09.2002 US 251111**

(43) Date of publication of application:
**22.06.2005 Bulletin 2005/25**

(73) Proprietor: **Abbott Cardiovascular Systems Inc.**
**Santa Clara, CA 95054 (US)**

(72) Inventors:
- **PACETTI, Stephen, D.**
  **San Jose, CA 95130 (US)**
- **HOSSAINY, Syed, F.A.**
  **Fremont, CA 94555 (US)**
- **DING, Ni**
  **San Jose, CA 95135 (US)**
- **ROORDA, Wouter, E.**
  **Palo Alto, CA 94306 (US)**

(74) Representative: **Keen, Celia Mary**
**J.A. Kemp & Co.**
**14 South Square**
**Gray's Inn**
**London WC1R 5JJ (GB)**

(56) References cited:
**WO-A-01/30403**
**WO-A-01/87368**
**WO-A-02/26271**
**US-A- 5 820 917**
**US-A- 5 837 008**
**US-A- 5 873 904**
**US-A- 5 879 697**
**US-A- 5 928 279**
**US-A- 6 153 252**
**US-A1- 2002 122 877**
**US-B1- 6 299 604**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 542 740 B1

## Description

BACKGROUND OF THE INVENTION

Field of the Invention

This invention relates to coatings for drug delivery devices, such as drug eluting vascular stents. More particularly, this invention is directed to coatings for controlling the rate of release of drugs from stents and methods of fabricating the same.

Description of Related Art

In the treatment of vascular disorders, stents have become a standard adjunct to balloon angioplasty. Stents can eliminate vasospasm, tack dissections to the vessel wall, and reduce negative remodeling. In addition to mechanical functionality, stents are being modified to provide pharmaceutical therapy. Local drug delivery with a stent can provide an efficacious concentration of a drug to the treatment site. In contrast, systemic administration of the medication may produce adverse or toxic side effects for the patient. Local delivery of a drug to the patient via a stent can be the preferred method of treatment in that smaller total levels of medication are administered in comparison to systemic dosages, but are concentrated at a specific site.

Stents are typically made from interconnected struts that are usually between 50 and 150 microns wide. Being made of a metal, such as stainless steel, bare stents have to be modified so as to provide a means for drug delivery. Accordingly, stents are being modified by forming a polymeric coating, containing a drug, on the surface of the stent. A polymer dissolved in a solvent and a drug added thereto can be sprayed on the stent or the stent can be immersed in the composition. Once the solvent evaporates from the composition, a polymeric film layer containing a drug remains on the surface of the stent.

To the extent that the mechanical functionality of stents has been optimized, continued improvements can be made to the coating for stents. For example, one improvement can be for maintaining the concentration of a drug at a therapeutically effective level for an acceptable period of time. Accordingly, controlling or, in effect, decreasing the rate of release of a drug from the stent is important in order to provide for long term sustained drug release. One way of controlling the release rate of the drug from a polymer layer is by the deposition of a topcoat layer on the drug-polymer layer. The topcoat layer serves as a barrier membrane, retarding the process of dissipation of the drug. The current topcoat technology can be improved by providing topcoats having low water absorption, high hydrophobicity and increased biological stability and compatibility. In addition, the topcoats can have other important functions, such as providing the stent with increased lubricity.

WO 02/26271 describes coated medical devices and sterilisation thereof. US 6,153,252 describes a process for coating stents. US 5,820,917 describes a blood-contacting medical device and method. US 5,837,008 describes an intravascular stent and method. US 2002/0122877 describes methods of forming a coating for a prosthesis. US 5,879,697 describes drug-releasing coatings for medical devices. US 5,928,279 describes stented, radially-expandable, tubular PTFE grafts. US 5,873,904 describes a silver implantable medical device. US 6,299,604 describes a coated implantable medical device. WO 01/30403 describes biocompatible medical devices. WO 01/87368 describes a process for coating medical devices using super-critical carbon dioxide.

In light of the foregoing, the embodiments of the present invention provide for coatings for implantable medical devices, such as stents, with improved characteristics for the delivery of pharmaceutical agents.

SUMMARY

The present invention provides a coating for an implantable medical devices, as defined in claim 1. The invention further provides a process for producing an implantable medical device which comprises a coating, as defined in claim 2. The invention further provides an implantable medical device as defined in claim 23. Examples of the fluorinated polymer include poly(vinylidene fluoride), poly(vinylidene fluoride-co-hexafluoropropene), poly(tetrafluoroethylene), fluorinated poly(ethylene-co-propylene), poly(hexafluoropropene), poly(chlorotrifluoroethylene), poly(vinylidene fluoride-co-tetrafluoroethylene), poly(tetrafluoroethylene-co-hexafluoropropene), poly(tetrafluoroethylene-co-vinyl alcohol), poly(tetrafluoroethylene-co-vinyl acetate), poly(tetrafluoroethylene-co-propene), poly(hexafluoropropene-co-vinyl alcohol), poly(tetrafluoroethylene-co-fluoromethylvinyl ether), poly(ethylene-co-tetrafluoroethylene), poly(ethylene-co-hexafluoropropene), poly(vinylidene

fluoride-co-chlorotrifluoroethylene), fluorinated silicones, and mixtures thereof. The fluorinated polymer can have a solubility parameter lower than about 11 $(cal/cm^3)^{1/2}$.

BRIEF DESCRIPTION OF THE DRAWING

FIGs. 1 and 2 illustrate the results of the drug release by coatings fabricated according to some embodiments of the present invention.

FIGs. 3-5 are histology slides showing the results of the biocompatibility studies of coatings fabricated according to some embodiments of the present invention.

DETAILED DESCRIPTION

A stent coating according to the present invention can include an optional primer layer, a drug-polymer layer, a topcoat layer, an optional intermediate membrane, and an optional finishing coat layer. The drug-polymer layer serves as a reservoir for the therapeutic substance. The primer layer can be used if there is a need to improve the adhesion of the stent coating to the bare surface of the stent, particularly when the drug in the coating may compromise the adhesion. Each of these layers can be formed by dissolving a polymer in a suitable solvent to be selected by those having ordinary skill in the art, followed by applying the solution to the stent, for example, by dipping, brushing, spraying, or other conventional coating methods.

A copolymer of ethylene and vinyl alcohol (EVAL) is one example of a polymer that can be used to fabricate the optional primer layer and/or the drug-polymer layer. EVAL has the general formula-$[CH_2\text{-}CH_2]_m\text{-}[CH_2\text{-}CH(OH)]_n$- EVAL is a product of hydrolysis of ethylene-vinyl acetate copolymers and may also be a terpolymer including up to 5 molar % units derived from styrene, propylene and other suitable unsaturated monomers. A brand of copolymer of ethylene and vinyl alcohol distributed commercially by Aldrich Chemical Co. of Milwaukee, Wisconsin, or manufactured by EVAL Company of America of Lisle, Illinois, can be used.

Alternatively, a block copolymer can be used to fabricate the optional primer layer and/or the drug-polymer layer. The block-copolymer is also called "a segmented copolymer." The term "block copolymer" is defined in accordance with the terminology used by the International Union of Pure and Applied Chemistry (IUPAC) and refers to a copolymer containing a linear arrangement of blocks. The block is defined as a portion of a polymer molecule in which the monomeric units have at least one constitutional or configurational feature absent from the adjacent portions.

For example, a block copolymer of A and B may be written as ...-A-A-A-B-B-B-.... The blocks of "A" and "B" can have the same or different number of units of "A" and "B." The blocks need not be linked on the ends, since the individual blocks are usually long enough to be considered polymers in their own right. The term copolymer is intended to broadly include two or more types of blocks such as tri-blocks.

Examples of block-copolymers that can be used include such classes of block copolymers as polyureas, polyurethanes, polyureaurethanes, for example, BIOMER, styrene-butadiene-styrene tri-block copolymers, styrene-isoprene-styrene tri-block copolymers, and styrene-ethylene/propylene-styrene tri-block copolymers. The polyurethanes that can be used include:

(a) polyurethanes having poly(dimethylsiloxane) soft segments, such as ELAST-EON;
(b) polyurethanes having polycarbonate soft segments, such as BIONATE;
(c) polyurethanes having polyether soft segments, such as PELLETHANE, TECOTHANE or TECOFLEX;
(d) polyurethanes with polyester soft segments; and
(e) polyurethanes with aliphatic soft segment.

BIOMER is a trade name of a poly(ether-urethane-urea) tri-block copolymer and is available from Johnson & Johnson Co. of New Brunswick, New Jersey.

ELAST-EON is a trade name of a product of co-polycondensation of an isocyanate-based component (the hard segment) and a hydrophobic polymeric component (the soft segment) and is available from AorTech Biomaterials Co. of Chatswood, Australia. With respect to one grade of ELAST-EON, the isocyanate-based component can be synthesized by reacting an aromatic diisocyanate, 4,4'-methylene-bisphenyl-diisocyanate (MDI) with butane-1,4-diol. The hydrophobic soft segment can be a blend of poly(hexamethylene glycol) and a carbinol-terminated polydimethylsiloxane (PDMS).

BIONATE is a trade name of a thermoplastic polycarbonate-urethane elastomer formed as the product of the reaction between a hydroxyl-terminated polycarbonate, an aromatic diisocyanate, and a low molecular weight glycol used as a chain extender. BIONATE is available from The Polymer Technology Group Incorporated of Berkeley, California.

PELLETHANE is a trade name of a family of polyether- or polyester-based thermoplastic polyurethane elastomers registered to Upjohn Co. of Kalamazoo, Michigan and available from Dow Chemical Co. of Midland, Michigan.

TECOTHANE is a trade name of a family of aromatic, polyether-based thermoplastic polyurethane elastomers

and TECOFLEX - a trade name of family of aliphatic, polyether-based thermoplastic polyurethane elastomers. Both TECOTHANE and TECOFLEX are available from Thermedics, Inc. of Woburn, Massachusetts.

Alternatively, the optional primer layer can be also fabricated of a silane, a siloxane, an amorphous fluorocarbon solvent-soluble perfluoropolymer, a fluorinated silicone, poly(vinylidene fluoride) (PVDF), a copolymer of poly(tetrafluoroethylene) (PTFE) and fluoromethylvinyl ether, a fluoroalkoxyl-containing polymer, a mixture of silicone and fluoropolymer, or combinations thereof.

Yet another example of a material suitable for making the optional primer layer is a PTFE/silicone copolymer, polymerized on the stent's surface via glow discharge. Still another example of a suitable polymer for fabricating the optional primer layer is a PARYLENE coating. PARYLENE is a trade name of a poly(*para*-xylylene)-based coating available from Specialty Coating Systems, Inc. of Indianapolis, Indiana.

If the adhesion still needs to be improved, a primer layer having more than one sub-layer can be used, e.g. poly(butyl methacrylate) sub-layer may be applied to the bare stent first, followed by application of a fluorine-containing polymer such as PTFE-co-fluoromethylvinyl ether, and finally followed by application of the amorphous PTFE.

Alternatively, other polymers can be used to make the optional primer layer and/or the drug-polymer layer, if desired. Representative examples of such alternative polymers include poly(amino acids), cyanoacrylates, poly(trimethylene carbonate), poly(iminocarbonate), copoly(ether-esters) (e.g. PEO/PLA), polyalkylene oxalates, polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers (such as polyvinyl methyl ether), polyvinylidene halides, such as polyvinylidene chloride, polyacrylonitrile, polyvinyl ketones, polyvinyl aromatics (such as polystyrene), polyvinyl esters (such as polyvinyl acetate), copolymers of vinyl monomers with each other and olefins (such as ethylene-methyl methacrylate copolymers, acrylonitrile-styrene copolymers, ABS resins, and ethylene-vinyl acetate copolymers), polyamides (such as Nylon 66 and polycaprolactam), alkyd resins, polycarbonates, polyoxymethylenes, polyimides, polyethers, epoxy resins, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose.

The therapeutic substance of drug can include any substance capable of exerting a therapeutic or prophylactic effect in the practice of the present invention. The drug may include small molecule drugs, peptides or proteins. The drug can be for inhibiting abnormal or inappropriate migration and proliferation of smooth muscular cells for the treatment of restenosis.

Examples of the drugs which are usable include antiproliferative substances such as actinomycin D, or derivatives and analogs thereof. Synonyms of actinomycin D include dactinomycin, actinomycin IV, actinomycin $I_1$, actinomycin $X_1$, and actinomycin $C_1$. The active agent can also fall under the genus of antineoplastic, anti-inflammatory, antiplatelet, anticoagulant, antifibrin, antithrombin, antimitotic, antibiotic, antiallergic and antioxidant substances. Examples of antineoplastics and/or antimitotics include paclitaxel, docetaxel, methotrexate, azathioprine, vincristine, vinblastine, fluorouracil, doxorubicin hydrochloride, and mitomycin. Examples of antiplatelets, anticoagulants, antifibrin, and antithrombins include sodium heparin, low molecular weight heparins, heparinoids, heparin derivatives containing hydrophobic counterions, hirudin, argatroban, forskolin, analogues, vapiprost, prostacyclin and prostacyclin dextran, D- phe-pro-arg-chloromethylketone (synthetic antithrombin), dipyridamole, glycoprotein IIb/IIIa platelet membrane receptor antagonist antibody, recombinant hirudin, and thrombin. Examples of cytostatic or antiproliferative agents include angiopeptin, angiotensin converting enzyme inhibitors such as captopril, cilazapril or lisinopril, calcium channel blockers (such as nifedipine), colchicine, fibroblast growth factor (FGF) antagonists, fish oil (ω-3-fatty acid), histamine antagonists, lovastatin (an inhibitor ofHMG-CoA reductase, a cholesterol lowering drug), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents which may be appropriate include alpha-interferon, genetically engineered epithelial cells, tacrolimus, clobetasol, dexamethasone and its derivatives, and rapamycin, its derivatives and analogs, such as 40-O-(2-hydroxy)ethyl-rapamycin (known by the trade name of EVEROLIMUS available from Novartis Corp. of New York), 40-O-(3-hydroxy)propyl-rapamycin, 40-O-[2-(2-hydroxy)ethoxy]ethyl-rapamycin, and 40-O-tetrazole-rapamycin.

EVAL can be also used to make the optional finishing coat layer. However, to provide a topcoat layer with improved barrier properties, it is desirable to choose a polymer other than EVAL. Thus, the topcoat layer and the optional finishing coat layer can be fabricated of a polymer having hydrophobicity higher than that of pure EVAL.

Generally, hydrophobicity of a polymer can be gauged using the Hildebrand solubility parameter $\delta$. Hydrophobic polymers typically have a low $\delta$ value. A polymer sufficiently hydrophobic to be used in the topcoat layer or the optional finishing coat layer can have a solubility parameter lower than about 11 $(cal/cm^3)^{1/2}$. The term "Hildebrand solubility parameter" refers to a parameter measuring the cohesive energy density of a substance. The $\delta$ parameter is determined as follows:

$$\delta = (\Delta E/V)^{1/2}$$

where $\delta$ is the solubility parameter, $(cal/cm^3)^{1/2}$; $\Delta E$ is the energy of vaporization, cal/mole; and V is the molar volume, $cm^3$/mole.

Consequently, various embodiments of the present invention described below are directed to the stent coating such that the outermost layer of the coating (i.e., the topcoat layer or the optional finishing coat layer) includes a hydrophobic fluorinated polymer soluble in an organic solvent or a blend of organic solvents. In some embodiments, more particularly in embodiments in which a topcoat layer as well as a finishing coat layer disposed on the topcoat layer is used, both the topcoat layer and finishing coat layer may include a fluorinated polymer.

Examples of highly fluorinated polymers include PVDF having a general formula $-[CF_2-CH_2]_m-$, and poly(vinylidene fluoride-co-hexafluoropropene) (PVDF-HFP) having a general formula

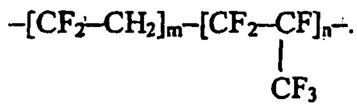

A brand of PVDF known under the trade name KYNAR available from Atofina Chemicals, Inc. of Philadelphia, Pennsylvania, can be used.

In the alternative, those having ordinary skill in the art may select other highly fluorinated polymers. For the purposes of the present invention, the term "highly fluorinated polymer" is defined as any homopolymer, copolymer, terpolymer or a blend thereof in which at least 50% of monovalent atoms in the macromolecule are fluorine atoms.

One group of such suitable alternative highly fluorinated polymers includes polymers based on fluorinated olefins or mixtures thereof. The term "polymers based on fluorinated olefins" refers to the polymers which include units derived from fully or partially fluorinated olefins, such as fluorinated ethylene. Examples of some polymers belonging to this group are provided in Table 1.

Table 1. Examples of Olefm-Based Fluorinated Polymers Suitable for Stent Coatings.

| No. | Fluorinated Polymer | Abbreviation | General Formula |
|---|---|---|---|
| 1 | Poly(tetrafluoroethylene) *) | PTFE | $-[CF_2-CF_2]_m-$ |
| 2 | Fluorinated poly(ethylene-co-propylene) | FPEP | 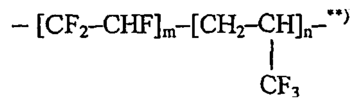 |
| 3 | Poly(hexafluoropropene) | PHFP | 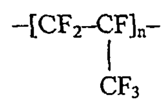 |
| 4 | Poly(chlorotrifluoroethylene) | PCTFE | $-[CClF-CF_2]_m-$ |
| 5 | Poly(vinylidene fluoride)***) | PVDF | $-[CF_2-CH_2]_m-$ |
| 6 | Poly(vinylidene fluoride-co-tetrafluoroethylene) | PVDF-TFE | $-[CF_2-CH_2]_m-[CF_2-CF_2]_n-$ |
| 7 | Poly(vinylidene fluoride-co-hexafluoropropene) | PVDF-HFP | 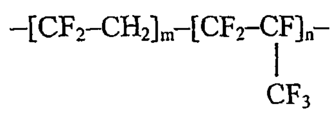 |
| 8 | Poly(tetrafluoroethylene-co-hexafluoropropene) | PTFE-HFP | 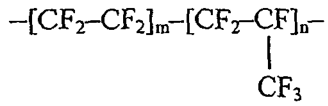 |

(continued)

| No. | Fluorinated Polymer | Abbreviation | General Formula |
|---|---|---|---|
| 9 | Poly(tetrafluoroethylene-co-vinyl alcohol) | PTFE-VAL | 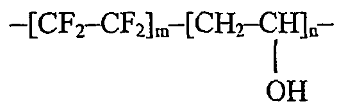 |
| 10 | Poly(tetrafluoroethylene-co-vinyl acetate) | PTFE-VAC | 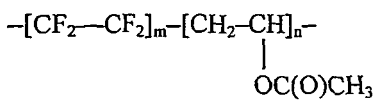 |
| 11 | Poly(tetrafluoroethylene-co-propene) | PTFEP | 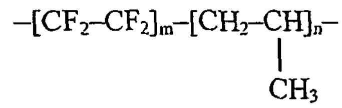 |
| 12 | Poly(hexafluoropropene-co-vinyl alcohol) | PHFP-VAL | 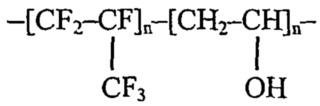 |
| 13 | Poly(ethylene-co- tetrafluoroethylene) | PETFE | $-[CH_2-CH_2]_m-[CF_2-CF_2]_n-$ |
| 14 | Poly(ethylene-co-hexafluoropropene) | PEHFP | 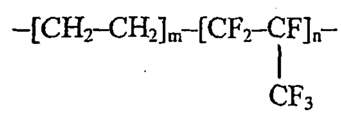 |
| 15 | Poly(vinylidene fluoride-co-chlorotrifluoroethylene) | PVDF-CTFE | $-[CF_2-CH_2]_m-[CClF-CF_2]_m-$ |

*) Including various brands of TEFLON available from E.I. DuPont de Nemours & Co. of Wilmington, Delaware.
**) The formula shows an example of one possible FPEP. Other kinds of FPEP can be used.
***)Including various brands of KYNAR

The fluorinated polymers discussed above are highly hydrophobic. For example, PTFE has a Hildebrand solubility parameter of 6.2. Other highly fluorinated polymers that can be used for making the topcoat layer and/or the finishing coat layer include polymers having heterocyclic fragments or having oxygen atoms in the backbone. These classes of polymers are not based on fluorinated olefins. Examples of such polymers include:

(1) amorphous products of polymerization of fluorinated cyclic esters, such as poly(perhalo-2,2-di-loweralkyl-1,3-dioxole-co-perfluoro-2-methylene-methyl-1,3-dioxolane) (designated for the purposes of this invention as "polyfluorooxalanes"), for example, poly(perhalo-2,2-dimethyl-1,3-dioxole-co-perfluoro-2-methylene-methyl-1,3-dioxolane);
(2) thermoplastic resinous fluorine-containing cyclic polymers having a main chain with an asymmetrical cyclic structure, with repeating units of cyclically polymerized perfluorallyl vinyl ether and/or perfluorobutenyl vinyl ether, e.g., poly(perfluorobutenyl vinyl ether) (PPBVE); and
(3) copolymers of perfluoro-2,2-dimethyl-1,3-dioxole (PDD) with such monomers as perfluoroolefins and perfluoro (alkyl vinyl) ethers (designated for the purposes of this invention as "polyfluorooxoles"), including the TEFLON AF product. TEFLON AF is a trade name of a product which includes poly(tetrafluoroethylene-co-perfluoro-2,2-dimethyl-1,3-dioxole) and which is available from E.I. DuPont de Nemours & Co.

Polyfluorooxoles can contain between about 1 and 99.5% (molar) units derived from PDD and the balance of units derived from perfluoro(butenyl vinyl ether), and can optionally contain minor amounts of additional monomers, such as chlorinated or fluorinated olefins, e.g., tetrafluoroethylene or chlorotrifluoroethylene, and perfluorvinyl ethers such as perfluoropropylvinyl ether, perfluoro-3,6-dioxa-4-methyl-7-octenesulfonyl fluoride and methyl perfluoro-4,7-dioxa-5-methyl-8-nonenoate. A PPVBE brand under the trade name CYTOP, available from Asahi Glass Co. of Charlotte, North Carolina, can be used.
All fluorinated polymers used in the present invention are soluble in at least one organic solvent, or a blend of various organic solvents. Suitable solvents include fluorinated solvents, for example, fluorocarbon systems having the

boiling temperature of about 60˚C to about 140˚C, such as FLUORINERT FC-75 and various FREONs, and other fluorinated solvents, such as FLUX REMOVER AMS and NOVEC hydrofluoroether solvents.

FLUORINERT FC-75 is a trade name of perfluoro(2-butyltetrahydrofuran), a solvent which is available from Minnesota Mining and Manufacturing Corp. of Saint Paul, Minnesota. FREON is a trade name of various chlorinated fluorocarbons available from E.I. DuPont de Nemours & Co.

FLUX REMOVER AMS is trade name of a solvent manufactured by Tech Spray, Inc. of Amarillo, Texas comprising about 93.7% of a mixture of 3,3-dichloro-1,1,1,2,2-pentafluoropropane and 1,3-dichloro-1,1,2,2,3-pentafluoropropane, and a balance of methanol, with trace amounts of nitromethane. NOVEC is a trade name of a family of solvents based on hydrofuoroethers available from 3M Corp. of St. Paul, Minnesota.

Other solvents can be alternatively used to dissolve the above described fluorinated polymers. Representative examples of such other suitable solvents include N,N-dimethylacetamide (DMAC), N,N-dimethylformamide (DMF), dimethylsulphoxide (DMSO), acetone, cyclohexanone, methyl isobutyl ketone, methyl ethyl ketone, N-methyl pyrrolidone, and 1,4-dioxane.

To form the topcoat layer, the finishing layer and/or the drug-polymer layer, the layer can be applied from a polymer solution as described above. To prepare the polymer solution, one or a blend of several of the fluoropolymers described above can be dissolved in one or a blend of several of the above-mentioned solvents. If it is desirable to incorporate EVAL or other non-fluorinated polymers described above into the topcoat layer and/or the finishing layer, they can be included in the polymer solution. No cross-linking of the coating or exposure of the coating to high temperatures is required for the curing of the coating, but moderate heat can be optionally applied to facilitate the removal of the solvent.

To improve the barrier properties of the topcoat layer even more, in one embodiment, an intermediate membrane can be applied below the topcoat layer, or between the topcoat layer and the finishing layer which is deposited on top of the topcoat layer. The intermediate membrane can be applied by chemical vapor deposition according to techniques known to those skilled in the art. Typical materials used for depositing the intermediate membrane include tetrafluoroethylene and vinylidene fluoride to obtain a PTFE-like or PVDF-like membrane.

Non-fluorinated materials, such as PARYLENE or DYLYN can alternatively be used to make the intermediate membrane. DYLYN is a trade name of a pyrolytic carbon coating having abstractable hydrogen (diamond-like coating having both $sp^2$ and $sp^3$ carbon atoms and applied by plasma-assisted chemical vapor deposition). DYLYN can be obtained from ART, Inc. of Buffalo, New York.

The coatings of all the embodiments of the present invention have been described in conjunction with a stent. However, the coatings can also be used with a variety of other medical devices. Examples of the implantable medical devices that can be used in conjunction with the embodiments of this invention, include stent-grafts, grafts (e.g., aortic grafts), artificial heart valves, cerebrospinal fluid shunts, pacemaker electrodes, axius coronary shunts and endocardial leads (e.g., FINELINE and ENDOTAK, available from Guidant Corporation). The underlying structure of the device can be of virtually any design. The device can be made of a metallic material or an alloy such as, but not limited to, cobalt-chromium alloys (e.g., ELGILOY), stainless steel (316L), "MP35N," "MP20N," ELASTINITE (Nitinol), tantalum, tantalum-based alloys, nickel-titanium alloy, platinum, platinum-based alloys such as, e.g., platinum-iridium alloy, iridium, gold, magnesium, titanium, titanium-based alloys, zirconium-based alloys, or combinations thereof. Devices made from bioabsorbable or biostable polymers can also be used with the embodiments of the present invention.

"MP35N" and "MP20N" are trade names for alloys of cobalt, nickel, chromium and molybdenum available from Standard Press Steel Co. of Jenkintown, Pennsylvania. "MP35N" consists of 35% cobalt, 35% nickel, 20% chromium, and 10% molybdenum. "MP20N" consists of 50% cobalt, 20% nickel, 20% chromium, and 10% molybdenum.

EXAMPLES

Embodiments of the present invention can be further illustrated by the following Examples.

Example 1

A first composition was prepared by mixing the following components:

(a) about 2.0 mass% of EVAL; and

(b) the balance, a mixture of solvents, DMAC and ethanol, in a ratio of DMAC to ethanol of about 70:30 by mass.

The first composition was applied onto the surface of a bare 13 mm TETRA stent (available from Guidant Corp.) by spraying and dried to form a primer layer. A spray coater having an EFD 7803 spray valve with 0.036 cm (0.014 inch) fan nozzle with a VALVEMATE 7040 control system, manufactured by EFD, Inc. of East Providence, Rhode Island was used. The fan nozzle was maintained at about 60˚C with a feed pressure of about 0.2 atm (about 3 psi) and

an atomization pressure of about 1.35 atm (about 20 psi). An average of about 19 micrograms (μg) per coating pass was applied and an average total of about 62 μg of the wet coating was applied.

The primer layer was baked at about 140˚C for about one hour, yielding a layer with an average total amount of solids of about 61 μg, corresponding to an average thickness on the stent of 0.65 μm. "Solids" means the amount of dry residue deposited on the stent after all volatile organic compounds (e.g., the solvent) have been removed.

The second composition was prepared by mixing the following components:

(c) about 2.0 mass% of EVAL
(d) about 0.7 mass % rapamycin; and
(e) the balance, a mixture of solvents, DMAC and ethanol, in a ratio of DMAC to ethanol of about 70:30 by mass.

A second composition was applied onto the dried primer layer to form a drug-polymer layer using the same spraying technique and equipment used for the primer layer. About 497 μg of the wet coating was applied, followed by drying at about 50˚C for about 2 hours. The total amount of solids of the drug-polymer layer was about 494 μg, corresponding to an average thickness on the stent of about 5.3 μm.

A third composition was prepared by mixing the following components:

(g) about 2.0 mass % of PVDF-HFP; and
(h) the balance, a mixture of solvents, cyclohexanone, acetone, and AMS FLUX REMOVER in a ratio of 25:50:25 by mass.

The third composition was applied onto the drug-polymer layer, to form a topcoat layer, using the same spraying technique and equipment used for applying the primer and drug-polymer layers. About 475 μg of wet coating was applied, followed by baking at about 50˚C for about 2 hours. The average total amount of solids of the topcoat layer was about 449 μg, corresponding to an average thickness on the stent of about 3.08 μm.

The properties of the coating obtained according to the procedure described above are summarized as shown in Table 2.

Table 2. The Properties of the Coating of Example 1.

| Layer of the Coating | Weight, μg | Average Thickness, μm |
|---|---|---|
| EVAL Primer | 61 ± 5 | 0.65 |
| Rapamycin/EV AL drug-polymer layer | 494 ± 21 | 5.3 |
| PVDF-HFP topcoat layer | 449 ± 10 | 3.08 |
| Overall coating | 1,004 ± 36 | 9.03 |

Comparative Example 2

A primer layer and a drug-polymer layer were formed on a stent as described in Example 1. A topcoat composition was prepared by mixing the following components:

(a) about 2.0 mass % of EVAL; and
(b) the balance, a mixture of solvents, DMAC and pentane, in a ratio of DMAC to pentane of about 80:20 by mass.

The topcoat composition was applied onto the drug-polymer layer, to form a topcoat layer, using the same spraying technique and equipment used for applying the primer and drug-polymer layers. About 348 μg of wet coating was applied, followed by baking at about 50˚C for about 2 hours. The average total amount of solids of the topcoat layer was about 295 μg, corresponding to an average thickness on the stent of about 3.16 μm.

Example 3

The stents coated according to Example 1 and Comparative Example 2 were assayed for total drug content by solvent extraction followed by analysis by HPLC. Six stents were used for each group. The average amount of the drug present based on the gravimetric weight of the drug/polymer layer was about 80% of the theoretical amount.

The stents also were assayed for drug release. Again, six stents were used for each group. The stents were immersed in stirred porcine serum at about 37˚C for about 24 hours to simulate an *in vivo* environment. The drug

remaining on the stent was assayed using the same total drug content assay. It was found that the three stents with the PVDF-HFP topcoat released an average of about 6.5% of the drug indicating a slow release rate. Similar stents with a 285 μg topcoat membrane layer of EVAL released an average of about 14.7% of the rapamycin in about 24 hours under the same conditions. The comparative results for the two groups are provided in Table 3.

Table 3. Comparative Results of the Drug Release Study

| Topcoat Layer of the Stent Coating | Average Theoretical Amount of Rapamycin | Actual Amount of Rapamycin, % of Theoretical Amount | Rapamycin Released in 24 hours, % |
|---|---|---|---|
| PVDF-HFP | 127 | 80.5 | 6.5 |
| EVAL | 128 | 80.1 | 14.7 |

The topcoat thicknesses of the PVDF-HFP in Example 1, and the EVAL in Comparative Example 2 are close at 3.08 and 3.16 μm, respectively. As seen from the results presented in Table 2, the fluoropolymer topcoat layer of the stent coating provides a substantial (over 55%) decrease in the drug release rate compared to an EVAL topcoat layer.

Example 4

A first composition was prepared by mixing the following components:

(a) about 2.67 g of a 15 mass % solution of EVAL in DMAC;
(b) about 0.20 g of 17-β-estradiol; and
(c) about 17.13 g of additional DMAC.

The first composition was applied onto a stent, to form a drug-polymer layer. About 323 μg of the wet coating was applied. The total amount of solids of the drug-polymer layer was about 316 μg, corresponding to a thickness of about 3.38 μm.

A second composition was prepared by mixing the following components:

(d) about 6.0 g of a 5 mass % solution of KYNAR-FLEX 2800 in acetone;
(e) about 1.65 g of additional acetone;
(f) about 3.675 g of cyclohexanone; and
(g) about 3.675 g of AMS FLUX REMOVER.

The second composition was applied by spraying using an EFD 7803 spray valve with 0.036 cm (0.014 inch) fan nozzle to form a topcoat layer followed by drying. The nozzle temperature was at ambient with a feed pressure of about 0.2 atm (3 psi) and an atomization pressure of about 1 atm (15 psi). The dryer temperature was at ambient with a dryer air pressure of about 2.7 atm (40 psi). An average of about 15 μg per coating pass was applied and an average total of about 461 μg of wet coating was applied. This topcoat was baked at about 50°C for about two hours yielding a total amount of solids of about 439 μg, corresponding to a thickness of about 3.0 μm.

Example 5

Three stents coated according to Example 4 were assayed for total drug content by solvent extraction followed by analysis by HPLC. The percent drug present, based on the weight of the drug/polymer layer was 92 ± 1.1 %. The three stents were also assayed for drug release. The stents were immersed in stirred porcine serum at about 37°C for about 24 hours to simulate an *in vivo* environment. It was found that the three stents released an average of about 2.5% of the drug indicating a slow release rate. Similar stents with a 300 μg topcoat layer of EVAL released 100% of the 17-β-estradiol in about 24 hours under the same conditions.

Example 6

A drug-polymer layer was applied onto a stent as described in Example 1, except 17-β-estradiol was used instead of rapamycin. A topcoat composition was prepared by mixing the following components:

(a) about 1.2 g of a 10 mass % solution of KYNAR-FLEX 2800 in acetone;

(b) about 1.89 g of additional acetone;
(c) about 5.94 g of cyclohexanone; and
(d) about 2.97 g of AMS FLUX REMOVER.

The topcoat composition was applied by spraying using an EFD 7803 spray valve with 0.036 cm (0.014 inch) fan nozzle to form a topcoat layer, followed by drying. The nozzle temperature was at ambient with a feed pressure of about 0.2 atm (3 psi) and an atomization pressure of about 1 atm (15 psi). The dryer temperature was at ambient with a dryer air pressure of about 2.7 atm (40 psi). An average of about 5 μg per coating pass was applied and an average total of about 60 μg of wet coating was applied. The topcoat layer was baked at about 50°C for two hours yielding a total amount of solids of about 55 μg, corresponding to a thickness of about 0.38 μm.

Three stents coated according to this example were tested for the drug release rate. The stents were immersed in individual, stirred vessels containing a phosphate-buffered saline solution which included about 1 mass % of sodium dodecyl sulfate. The buffer solution had pH of about 7.4 thermostated at 37°C. The amount of 17-β-estradiol released was determined at measured intervals of time by HPLC. The percent drug released as a function of time for three stents is shown by FIG. 1. The data demonstrates good reproducibility. There is an initial small burst of drug during the first 20 hours, after which the release rate is approximately linear.

Example 7

A first composition was prepared by mixing the following components:

(a) about 10 g of a 10 mass % solution of EVAL in DMAC;
(b) about 0.8 g of EVEROLIMUS;
(c) about 9.56 g of additional DMAC; and
(d) 4.64 g of pentane.

The first composition was applied onto the surface of a bare 18 mm medium VISION stent using an EFD 780S spray valve with a 0.036 cm (0.014 inch) nozzle tip and a 0.071 (0.028 inch) round air cap to form a drug-polymer layer, followed by drying. The nozzle temperature was at about 45°C with a feed pressure of about 0.2 atm (3 psi) and an atomization pressure of about 1.3 atm (20 psi). The dryer temperature was 80°C with a dryer air pressure of about 1.3 atm (20 psi). An average of about 30 μg per coating pass was applied and an average total of about 332 μg of wet coating was applied. The drug-polymer layer was baked at about 80°C for about two hours yielding a total amount of solids of about 309 μg, corresponding to a thickness of about 2.1 μm.

A second composition was prepared by mixing the following components:

(e) about 4.0 g of a 10 mass % solution of KYNAR-FLEX 2800 in acetone;
(f) about 1.3 g of additional acetone;
(g) about 9.8 g of cyclohexanone; and
(h) about 4.9 g of AMS FLUX REMOVER.

The second composition was applied by spraying using an EFD 7803 spray valve with 0.036 cm (0.014 inch) fan nozzle tip and 0.036 cm (0.014 inch) fan air cap to form a topcoat layer, followed by drying. The nozzle temperature was at ambient with a feed pressure of about 0.2 atm (3 psi) and an atomization pressure of about 1 atm (15 psi). The dryer temperature was at ambient with a dryer air pressure of about 1.35 atm (20 psi). An average of about 10 μg per coating pass was applied. On one group of stents, an average weight of the wet topcoat layer was about 105 μg. On another group of stents, an average weight of the wet topcoat layer was about 164 μg. The topcoat layers in both cases were baked at about 80°C for about one hour yielding total amount of solids of about 79 and 131 μg, respectively, corresponding to average dry topcoat layer thicknesses of about 0.39 and 0.65 μm, respectively.

Three stents of each group were assayed for *in vitro* drug release. The stents were agitated at 37°C in a buffer solution, and at measured intervals of time each solution was assayed for drug content by HPLC. The fraction of EVEROLIMUS released as a function of time for the six stents (two groups of three stents each) is shown by FIG. 2.

In FIG. 2, curves 1-3 correspond to stents having 0.65 μm thick KYNAR-FLEX 2800 topcoat layer. Curves 4-6 correspond to stents having 0.39 μm thick KYNAR-FLEX 2800 topcoat layer. Curves 7-9 correspond to stents having no topcoat layer. FIG. 2 demonstrates that compared to the stents with no topcoat layers, stents having KYNAR-FLEX 2800 substantially reduce the rate of release of everolimus. Different thicknesses of the KYNAR-FLEX 2800 topcoat layer allow for different controlled release rates of EVEROLIMUS.

Example 8

In order to assess the chronic vascular response, a study was done to compare bare metal (uncoated) stents to stents coated KYNAR-FLEX 2800. Both coated and uncoated stents were implanted for 28 days in the porcine coronary system.

To make the coated stents, a first composition was prepared by mixing the following components:

(a) about 6.0 g of a 10 mass % solution of EVAL in DMAC;
(b) about 6.12 g of additional DMAC; and
(c) about 2.88 g of pentane.

The first composition was applied onto a stent using equipment and technique described in Example 1, to form a primer layer. About 66 μg of the wet coating was applied, followed by baking at about 140˚C for one hour. The total amount of solids of the dry primer layer was about 65 μg, corresponding to an average thickness of about 0.7 μm.

A second composition was prepared by mixing the following components:

(d) about 7.94 g of a 6.3 mass % solution of PVDF-HFP in acetone;
(e) about 12.25 g of cyclohexanone; and
(f) about 4.81 g of AMS FLUX REMOVER.

The second composition was applied by spraying using an EFD 7803 spray valve with 0.036 cm (0.014 inch) fan nozzle tip and 0.036 cm (0.014 inch) fan air cap to form a topcoat layer, followed by drying. The nozzle temperature was at ambient with a feed pressure of about 0.2 atm (3 psi) and an atomization pressure of about 1 atm (15 psi). The dryer temperature was at about 60˚C with a dryer air pressure of about 1.35 atm (20 psi). An average of about 20 μg per coating pass was applied. The number of passes was varied. The topcoat layer was baked at about 60˚C for about two hours. For one group of stents, the total amount of solids was about 200 μg, corresponding to average dry topcoat layer thicknesses of about 1.4 μm. For another group of stents, the total amount of solids was about 486 μg, corresponding to average dry topcoat layer thicknesses of about 3.3 μm. The stents of both groups coated as described above were mounted onto 3.0 x 13 mm TETRA catheters and sterilized by electron beam radiation.

Non-atherosclerotic healthy farm pigs of either sex, in the weight range of 30-40 kg were used. Seven animals were used with three stents implanted per animal. Ticlopidine, 500 mg, and Aspirin, 325 mg were administered daily starting one day prior to stent implantation. The coronary vessels were randomized. Nine coated stents having a thickness of the topcoat layer of about 3.3 μm, six coated stents having a thickness of the topcoat layer of about 1.4 μm, and six bare metal stent (controls) were used. The stents were implanted at a target stent-to-artery ratio of 1.1 to 1 (the diameter of the stents was about 10% bigger than the diameter of the arteries).

Of the seven swine, one animal expired 4 days post surgery. The rest of the animals were sacrificed at a 28 day time point post surgery. The vessels were explanted, preserved in 10% formalin, embedded in methacrylate resin, and stained with hemotoxilin and eosin dye. Histological sections were performed and photomicrographs were prepared. The histology slides are shown by FIG. 3 (for the stent having 1.4 μm-thick PVDF-HFP coating), FIG. 4 (3.3 μm-thick PVDF-HFP coating), and FIG. 5 (control bare stent). Morphometric analysis (microscopic examination) of the histograms was done using computerized planimetry. Vessel injury scoring was performed as described in R.S. Schwartz et al, Restenosis and the Proportional Neointimal Response to Coronary Artery Injury: Results in a Porcine Model, Journal of American College of Cardiology, vol. 19, pp. 267-274 (1992). The average vessel injury scores, percent area stenosis (the ratio between the area of neointima and the area circumscribed by inner elastic lamina), and neointimal thickness over the struts (reflecting the growth of the tissue over the stent struts) are shown in Table 3.

Table 3. A Summary of Experiments on Swine

| Treatment | Injury Score*) (the Schwartz method) | NeointimalThickness (mm) | Stenosis, % |
|---|---|---|---|
| Bare Stent, 5 stents averages | 1.34 ± 0.36 | 0.30 ± 0.18 | 32.6 ± 16.1 |
| 1.4 μm PVDF-HFP 5 stents averages | 1.13 ± 0.10 | 0.11 ± 0.04 | 15.3 ± 4.6 |

(continued)

| Treatment | Injury Score*) (the Schwartz method) | NeointimalThickness (mm) | Stenosis, % |
|---|---|---|---|
| 3.3 μm PVDF-HFP 8 stents averages | 1.18 ± 0.17 | 0.16 ± 0.11 | 23.5 ± 11.6 |
| *) The score of "0" (the lowest) indicates no injury; the score of "3" indicates the highest degree of injury. | | | |

The 28 days *in vivo* implantation of PVDF-HFP coated stents were well tolerated in the porcine model. No filling defects, lumenal narrowing, aneurysms or thrombus were noted upon angiographic and morphometric analysis. For all of the stents, the struts were well apposed to the vessel wall. The mean morphometric percent stenosis of the PVDF-HFP coated stents is at least equivalent to that of bare stainless steel, indicating suitable biocompatibility of PVDF-HFP polymer for use as a coronary stent coating.

<u>Example 9</u>

A first composition can be prepared by mixing the following components:

(a) between about 0.1 mass % and about 15 mass %, for example, about 2.0 mass % of EVAL;
(b) between about 0.05 mass % and about 1.0 mass %, for example, about 0.7 mass % of actinomycin D (AcD); and
(c) the balance, DMAC solvent.

The first composition can be applied onto a stent, to form a drug-polymer layer with about 40 μg of total solids, with or without the optional primer layer.

A second composition can be prepared by mixing the following components:

(d) between about 0.1 mass % and about 15 mass %, for example, about 1.5 mass % of PVDF; and

(e) the balance, DMAC solvent.

The second composition can be applied onto the dried drug-polymer layer, for example, by spraying or dipping, to form the topcoat layer. The topcoat layer can have, for example, a total solids weight of about 30 μg.

<u>Example 10</u>

A first composition can be prepared by mixing the following components:

(a) between about 0.1 mass % and about 15 mass %, for example, about 2.0 mass % of EVAL;
(b) between about 0.05 mass % and about 1.0 mass %, for example, about 0.7 mass % of AcD; and
(c) the balance, DMAC solvent.

The first composition can be applied onto a stent, to form a drug-polymer layer with about 40 μg of total solids, with or without the optional primer layer.

A second composition can be prepared by mixing the following components:

(d) between about 0.1 mass % and about 15 mass %, for example, about 1.5 mass % of PVDF; and

(e) the balance DMAC solvent.

The second composition can be applied onto the dried drug-polymer layer, for example, by spraying or dipping, to form a topcoat layer. The topcoat layer can have, for example, a total solids weight of about 30 μg.

A third composition can be prepared by mixing the following components:

(f) about 2.0 mass % of EVAL; and
(g) the balance, DMAC solvent.

The third composition can be applied onto the dried topcoat layer, to form a finishing layer. The finishing layer

can have, for example, a total solids weight of about 30 μg.

Example 11

A first composition can be prepared by mixing the following components:

(a) between about 0.1 mass % and about 15 mass %, for example, about 2.0 mass % of EVAL;
(b) between about 0.05 mass % and about 1.0 mass %, for example, about 0.7 mass % of AcD; and
(c) the balance, DMAC solvent.

The first composition can be applied onto a stent, to form a drug-polymer layer with about 40 μg of total solids, with or without the optional primer layer.
A second composition can be prepared by mixing the following components:

(d) between about 0.1 mass % and about 15 mass %, for example, about 1.77 mass % of PVDF-HFP;
(e) between about 0.1 mass % and about 15 mass %, for example, about 3.23 mass % of EVAL; and
(f) the balance, DMAC solvent.

The second composition can be applied onto the dried drug-polymer layer, for example, by spraying or dipping, to form the topcoat layer. The topcoat layer can have, for example, a total solids weight of about 30 μg.

Example 12

A first composition can be prepared by mixing the following components:

(a) between about 0.1 mass % and about 15 mass %, for example, about 2.0 mass % of EVAL;
(b) between about 0.05 mass % and about 1.0 mass %, for example, about 0.7 mass % of AcD; and
(c) the balance, DMAC solvent.

The first composition can be applied onto a stent, to form a drug-polymer layer with about 40 μg of total solids, with or without the optional primer layer.
A second composition can be prepared by mixing the following components:

(d) between about 0.1 mass % and about 15 mass %, for example, about 2.99 mass % of PVDF;
(e) between about 0.1 mass % and about 15 mass %, for example, about 1.58 mass % of EVAL; and
(f) the balance, DMAC solvent.

The second composition can be applied onto the dried drug-polymer layer, for example, by spraying or dipping, to form the topcoat layer. The topcoat layer can have, for example, a total solids weight of about 30 μg.

Example 13

A first composition can be prepared by mixing the following components:

(a) between about 0.1 mass % and about 15 mass %, for example, about 2.0 mass % of EVAL;

(b) between about 0.05 mass % and about 1.0 mass %, for example, about 0.7 mass % of AcD; and

(c) the balance, DMAC solvent.

The first composition can be applied onto a stent, to form a drug-polymer layer with about 40 μg of total solids, with or without the optional primer layer. A membrane based on a PTFE-like polymer can be formed on top of the drug-polymer layer by chemical vapor deposition of poly(tetrafluoro ethylene). The method of chemical vapor deposition is known to those having ordinary skill in the art. The membrane can have thickness between about 0.05 μm and about 0.25 μm, for example, about 0.1 μm.
A second composition can be prepared by mixing the following components:

(d) between about 0.1 mass % and about 15 mass %, for example, about 1.5 mass % of PVDF; and

(e) the balance, DMAC solvent.

The second composition can be applied onto the membrane fabricated by chemical vapor deposition as described above, for example, by spraying or dipping, to form the topcoat layer. The topcoat layer can have, for example, a total solids weight of about 30 μg.

Example 14

A first composition can be prepared by mixing the following components:

(a) between about 0.1 mass % and about 15 mass %, for example, about 2.0 mass % of ELAST-EON 55 D; and
(b) the balance, a mixture of solvents, DMAC and FLUX REMOVER AMS, in a ratio of DMAC to FLUX REMOVER AMS of about 50:50 by mass.

ELAST-EON 55 D is one of the polymers of the ELAST-EON family and is a an aromatic polyurethane based on a soft segment containing a carbinol-terminated siloxane.
The first composition can be applied onto the surface of a bare 13 mm TETRA stent by spraying and dried to form a primer layer. An average of between about 9 and 12 μg per coating pass can be applied and an average a total of about 50 μg of the wet coating can be applied. The first composition can be baked at about 100˚C for about 1 hour, yielding a primer layer.
A second composition can be prepared by mixing the following components:

(c) between about 0.1 mass % and about 15 mass %, for example, about 2.0 mass % of ELAST-EON 55 D;

(d) between about 0.1 mass % and 2.0 mass %, for example, 0.7 mass % of EVEROLIMUS; and

(e) the balance, a mixture of solvents, DMAC and FLUX REMOVER AMS, in a ratio of DMAC to FLUX REMOVER AMS of about 50:50 by mass.

The second composition is applied on top of the dried primer layer to form the drug-polymer layer. The method of applying of the second composition can be the same as for the first composition. An average of between about 14 and 24 μg per coating pass can be applied. After the second composition is applied, it can be baked at about 60˚C for about 2 hours, to yield, for example, between about 294 and 311 μg of the dried drug-polymer layer.
A third composition can be prepared by mixing the following components:

(f) between about 0.1 mass % and about 15 mass %, for example, about 2.0 mass % of PVDF-HFP; and

(g) the balance a mixture of solvents, DMAC and acetone, in a ratio of DMAC to acetone of about 50:50 by mass.

The third composition can be applied onto the dried drug-polymer layer, for example, by spraying or dipping, to form the topcoat layer. An average of between about 16 and 19 μg per coating pass can be applied. After the third composition is applied, it can be baked at about 60˚C for about 2 hours, to yield, for example, between about 275 and 300 μg of the dried topcoat layer.

## Claims

**1.** A coating for an implantable medical device, which coating comprises:

(a) a drug-polymer layer, which drug-polymer layer comprises a therapeutic substance and a first polymer, which first polymer is selected from a copolymer of ethylene and vinyl alcohol (EVAL), a polyurea block copolymer, a styrene-butadiene-styrene tri-block copolymer, a styrene-isoprene-styrene tri-block copolymer, a polyimide, a polyether, an epoxy resin, rayon, rayon-triacetate, cellulose acetate, cellulose butyrate, cellulose acetate butyrate, cellophane, cellulose nitrate, cellulose propionate, a cellulose ether, carboxymethyl cellulose, a poly (amino acid), a cyanoacrylate, a poly(trimethylene carbonate), a poly(iminocarbonate), a copoly(ether-ester), a polyalkylene oxalate, a polyphosphazene, fibrin, fibrinogen, cellulose, starch, collagen, hyaluronic acid, a polyurethane, a silicone, a polyester, a polyisobutylene and ethylene-alphaolefin copolymer, an acrylic polymer or copolymer, a polyvinyl ester, polyvinylidene chloride, polyacrylonitrile, a polyvinylketone, a polyvinyl aromatic,

a polyamide, an alkyd resin, a polycarbonate and a polyoxymethylene; and
(b) a topcoat layer, which topcoat layer comprises a second, fluorinated polymer, which fluorinated polymer is soluble in an organic solvent or a mixture of organic solvents.

**2.** A process for producing an implantable medical device which comprises a coating, which coating comprises:

(a) a drug-polymer layer which comprises a therapeutic substance and a first polymer, which first polymer is selected from a copolymer of ethylene and vinyl alcohol (EVAL), a polyurea block copolymer, a styrene-butadiene-styrene tri-block copolymer, a styrene-isoprene-styrene tri-block copolymer, a polyimide, a polyether, an epoxy resin, rayon, rayon-triacetate, cellulose acetate, cellulose butyrate, cellulose acetate butyrate, cellophane, cellulose nitrate, cellulose propionate, a cellulose ether, carboxymethyl cellulose, a poly(amino acid), a cyanoacrylate, a poly(trimethylene carbonate), a poly(iminocarbonate), a co-poly(ether-ester), a polyalkylene oxalate, a polyphosphazene, fibrin, fibrinogen, cellulose, starch, collagen, hyaluronic acid, a polyurethane, a silicone, a polyester, a polyisobutylene and ethylene-alphaolefin copolymer, an acrylic polymer or copolymer, a polyvinyl ester, polyvinylidene chloride, polyacrylonitrile, a polyvinylketone, a polyvinyl aromatic, a polyamide, an alkyd resin, a polycarbonate and a polyoxymethylene, and
(b) a topcoat layer which comprises a second, fluorinated polymer, which fluorinated polymer is soluble in an organic solvent or a mixture of organic solvents, which process comprises:

(i) forming said drug-polymer layer by dissolving said first polymer and said therapeutic substance in a first solvent and applying the resulting solution to an implantable medical device; and
(ii) forming said topcoat layer by applying a solution of said second, fluorinated polymer in said organic solvent or mixture of organic solvents to the implantable medical device and allowing the organic solvent or solvents to evaporate.

**3.** A coating according to claim 1 or a process according to claim 2 wherein the device is a stent.

**4.** A coating according to claim 1 or claim 3 or a process according to claim 2 or claim 3, wherein the fluorinated polymer is an olefin-based polymer.

**5.** A coating according to claim 1, 3 or 4 or a process according to any one of claims 2 to 4, wherein the fluorinated polymer is poly(vinylidene fluoride), poly(vinylidene fluoride-co-hexafluoropropene), poly(tetrafluoroethylene), fluorinated poly(ethylene-co-propylene), poly(hexafluoropropene), poly(chlorotrifluoroethylene), poly(vinylidene fluoride-co-tetrafluoroethylene), poly(tetrafluoroethylene-co-hexafluoropropene), poly(tetrafluoroethylene-co-vinyl alcohol), poly(tetrafluoroethylene-co-vinyl acetate), poly(tetrafluoroethylene-co-propene), poly(hexafluoropropene-co-vinyl alcohol), poly(tetrafluoroethylene-co-fluoromethylvinyl ether), poly(ethylene-co-tetrafluoroethylene), poly(ethylene-co-hexafluoropropene), poly(vinylidenefluoride-co-chlorotrifluoroethylene), fluorinated silicones, or mixtures thereof.

**6.** A coating according to any one of claims 1 and 3 to 5 or a process according to any one of claims 2 to 5, wherein the fluorinated polymer has a solubility parameter lower than 11 $(cal/cm^3)^{1/2}$.

**7.** A coating according to any one of claims 1 and 3 to 6 or a process according to any one of claims 2 to 6 wherein the fluorinated polymer comprises units derived from fluorinated cyclic esters.

**8.** A coating or process according to claim 7, wherein the fluorinated polymer comprises poly(perhalo-2,2-dimethyl-1,3-dioxole-co-perfluoro-2-methylene-methyl-1,3-dioxolane), poly(perfluoroolefin-co-perfluoro-2,2-dimethyl-1,3-dioxole), or poly[perfluoro(alkyl vinyl)ether-co-perfluoro-2,2-dimethyl-1,3-dioxole].

**9.** A coating or process according to claim 8, wherein poly (perfluoroolefin-co-perfluoro-2,2-dimethyl-1,3-dioxole) is poly(tetrafluoroethylene-co-perfluoro-2,2-dimethyl-1,3-dioxole).

**10.** A coating according to any one of claims 1 and 3 to 9 or a process according to any one of claims 2 to 9 wherein the fluorinated polymer comprises units derived from fluorinated vinyl ethers.

**11.** A coating or process according to claim 10, wherein the fluorinated polymer is poly(perfluorobutenyl vinyl ether).

**12.** A coating according to any one of claims 1 and 3 to 11 or a process according to any one of claims 2 to 11, wherein

the organic solvent or mixture of organic solvents is a fluorinated organic substance or a mixture of fluorinated organic substances.

**13.** A coating according to any one of claims 1 and 3 to 12 or a process according to any one of claims 2 to 12 wherein the organic solvent or mixture of organic solvents has a boiling temperature between 60 ˚C and 140 ˚C.

**14.** A coating according to any one of claims 1 and 3 to 13 or a process according to any one of claims 2 to 13 wherein the organic solvent is perfluoro (2-butyltetrahydrofuran) or a chlorinated fluorocarbon.

**15.** A coating according to any one of claims 1 and 3 to 14 or a process according to any one of claims 2 to 14 wherein the organic solvent or mixture of organic solvents comprises a mixture of 3,3-dichloro-1,1,1,2,2-pentafluoropropane and 1,3-dichloro-1,1,2,2,3-pentafluoropropane.

**16.** A coating according to any one of claims 1 and 3 to 15 or a process according to any one of claims 2 to 15, wherein the organic solvent or mixture of organic solvents is N,N-dimethylacetamide, N,N-dimethylformamide, dimethylsulfoxide, acetone, cyclohexanone, methyl isobutyl ketone, methyl ethyl ketone, N-methyl pyrrolidone, 1,4-dioxane or a mixture thereof.

**17.** A coating according to any one of claims 2 to 16, which further comprises a pyrolytic carbon-based polymer or poly (para-xylylene).

**18.** A coating According to any one of claims 1 and 3 to 17 or a process according to any one of claims 2 to 16, wherein the therapeutic substance is rapamycin, 40-O-(2-hydroxy)ethyl-rapamycin, 40-O-[2-(2-hydroxy)ethoxy]ethyl-rapamycin, 40-O-(3-hydroxy)propyl-rapamycin or 40-O-tetrazole-rapamycin.

**19.** A coating according to any one of claims 1 and 3 to 18, further comprising a block copolymer.

**20.** A coating according to claim 19, wherein the block copolymer is a polyurea, a polyurethane, a polyureaurethane, a styrene-butadiene-styrene tri-block copolymer, a styrene-isoprene-styrene tri-block copolymer, or a styrene-ethylene/propylene-styrene tri-block copolymer.

**21.** A process according to any one of claims 2 to 16 and 18, further comprising including into the coating a pyrolytic carbon-based polymer or poly(para-xylylene).

**22.** A coating according to any one of claims 1 and 3 to 20 or a process according to any one of claims 2 to 16, 18 and 21 wherein the first polymer is a copolymer of ethylene and vinyl alcohol (EVAL).

**23.** An implantable medical device which comprises a coating as defined in any one of claims 1, 3 to 20 and 22.

**Patentansprüche**

**1.** Beschichtung für eine implantierbare medizinische Vorrichtung, wobei die Beschichtung umfasst:

(a) eine Arzneistoff-Polymer-Schicht, wobei die Arzneistoff-Polymer-Schicht eine therapeutische Substanz und ein erstes Polymer umfasst, wobei das erste Polymer aus einem Copolymer aus Ethylen und Vinylalkohol (EVAL), einem Polyharnstoff-Blockcopolymer, einem Styrol-Butadien-Styrol-Triblockcopolymer, einem Styrol-Isopren-Styrol-Triblockcopolymer, einem Polyimid, einem Polyether, einem Epoxyharz, Reyon, Reyon-Triacetat, Celluloseacetat, Cellulosebutyrat, Celluloseacetatbutyrat, Cellophan, Cellulosenitrat, Cellulosepropionat, einem Celluloseether, Carboxymethylcellulose, einer Poly(aminosäure), einem Cyanacrylat, einem Poly(trimethylencarbonat), einem Poly(iminocarbonat), einem Copoly(ether-ester), einem Polyalkylenoxalat, einem Polyphosphazen, Fibrin, Fibrinogen, Cellulose, Stärke, Kollagen, Hyaluronsäure, einem Polyurethan, einem Silikon, einem Polyester, einem Polyisobutylen und einem Ethylen-alpha-Olefin-Copolymer, einem Acrylpolymer oder -copolymer, einem Polyvinylester, Polyvinylidenchlorid, Polyacrylnitril, einem Polyvinylketon, einer aromatischen Polyvinylverbindung, einem Polyamid, einem Alkydharz, einem Polycarbonat und einem Polyoxymethylen ausgewählt ist, und
(b) eine Deckschicht, wobei die Deckschicht ein zweites, fluoriertes Polymer umfasst, wobei das fluorierte Polymer in einem organischen Lösungsmittel oder einem Gemisch von organischen Lösungsmitteln löslich ist.

2. Verfahren zur Herstellung einer implantierbaren medizinischen Vorrichtung, die eine Beschichtung umfasst, wobei die Beschichtung umfasst:

(a) eine Arzneistoff-Polymer-Schicht, die eine therapeutische Substanz und ein erstes Polymer umfasst, wobei das erste Polymer aus einem Copolymer aus Ethylen und Vinylalkohol (EVAL), einem Polyharnstoff-Blockcopolymer, einem Styrol-Butadien-Styrol-Triblockcopolymer, einem Styrol-Isopren-Styrol-Triblockcopolymer, einem Polyimid, einem Polyether, einem Epoxyharz, Reyon, Reyon-Triacetat, Celluloseacetat, Cellulosebutyrat, Celluloseacetatbutyrat, Cellophan, Cellulosenitrat, Cellulosepropionat, einem Celluloseether, Carboxymethylcellulose, einer Poly(aminosäure), einem Cyanacrylat, einem Poly(trimethylencarbonat), einem Poly(iminocarbonat), einem Copoly(ether-ester), einem Polyalkylenoxalat, einem Polyphosphazen, Fibrin, Fibrinogen, Cellulose, Stärke, Kollagen, Hyaluronsäure, einem Polyurethan, einem Silikon, einem Polyester, einem Polyisobutylen und einem Ethylen-alpha-Olefin-Copolymer, einem Acrylpolymer oder -copolymer, einem Polyvinylester, Polyvinylidenchlorid, Polyacrylnitril, einem Polyvinylketon, einer aromatischen Polyvinylverbindung, einem Polyamid, einem Alkydharz, einem Polycarbonat und einem Polyoxymethylen ausgewählt ist, und
(b) eine Deckschicht, die ein zweites, fluoriertes Polymer umfasst, wobei das fluorierte Polymer in einem organischen Lösungsmittel oder einem Gemisch von organischen Lösungsmitteln löslich ist, wobei das Verfahren umfasst:

(i) Bilden der Arzneistoff-Polymer-Schicht durch Lösen des ersten Polymers und der therapeutischen Substanz in einem ersten Lösungsmittel und Aufbringen der resultierenden Lösung auf eine implantierbare medizinische Vorrichtung, und
(ii) Bilden der Deckschicht durch Aufbringen einer Lösung des zweiten, fluorierten Polymers in dem organischen Lösungsmittel oder dem Gemisch von organischen Lösungsmitteln auf die implantierbare medizinische Vorrichtung und Verdampfenlassen des organischen Lösungsmittels oder der organischen Lösungsmittel.

3. Beschichtung nach Anspruch 1 oder Verfahren nach Anspruch 2, wobei die Vorrichtung ein Stent ist.

4. Beschichtung nach Anspruch 1 oder Anspruch 3 oder Verfahren nach Anspruch 2 oder Anspruch 3, wobei das fluorierte Polymer ein Polymer auf Olefinbasis ist.

5. Beschichtung nach Anspruch 1, 3 oder 4 oder Verfahren nach einem der Ansprüche 2 bis 4, wobei es sich bei dem fluorierten Polymer um Poly(vinylidenfluorid), Poly(vinylidenfluorid-co-hexafluorpropen), Poly(tetrafluorethylen), fluoriertes Poly(ethylen-co-propylen), Poly(hexafluorpropen), Poly(chlortrifluorethylen), Poly(vinylidenfluorid-co-tetrafluorethylen), Poly(tetrafluorethylen-co-hexafluorpropen), Poly(tetrafluorethylen-co-vinylalkohol), Poly(tetrafluorethylen-co-vinylacetat), Poly(tetrafluorethylen-co-propen), Po-ly(hexafluorpropen-co-vinylalkohol), Poly(tetrafluorethylen-co-fluormethylvinylether), Poly(ethylen-co-tetrafluorethylen), Poly(ethylen-co-hexafluorpropen), Poly(vinylidenfluorid-co-chlortrifluorethylen), fluorierte Silikone oder Gemische davon handelt.

6. Beschichtung nach einem der Ansprüche 1 und 3 bis 5 oder Verfahren nach einem der Ansprüche 2 bis 5, wobei das fluorierte Polymer einen Löslichkeitsparameter von weniger als 11 $(cal/cm^3)^{1/2}$ aufweist.

7. Beschichtung nach einem der Ansprüche 1 und 3 bis 6 oder Verfahren nach einem der Ansprüche 2 bis 6, wobei das fluorierte Polymer Einheiten umfasst, die von fluorierten cyclischen Estern abgeleitet sind.

8. Beschichtung oder Verfahren nach Anspruch 7, wobei das fluorierte Polymer Poly(perhalo-2,2-dimethyl-1,3-dioxol-co-perfluor-2-methylenmethyl-1,3-dioxolan), Poly(perfluorolefin-co-perfluor-2,2-dimethyl-1,3-dioxol) oder Poly[perfluor(alkylvinyl)ether-co-perfluor-2,2-dimethyl-1,3-dioxol] umfasst.

9. Beschichtung oder Verfahren nach Anspruch 8, wobei das Poly(perfluorolefin-co-perfluor-2,2-dimethyl-1,3-dioxol) Poly(tetrafluorethylen-co-perfluor-2,2-dimethyl-1,3-dioxol) ist.

10. Beschichtung nach einem der Ansprüche 1 und 3 bis 9 oder Verfahren nach einem der Ansprüche 2 bis 9, wobei das fluorierte Polymer Einheiten umfasst, die von fluorierten Vinylethern abgeleitet sind.

11. Beschichtung oder Verfahren nach Anspruch 10, wobei das fluorierte Polymer Po-ly(perfluorbutenylvinylether) ist.

12. Beschichtung nach einem der Ansprüche 1 und 3 bis 11 oder Verfahren nach einem der Ansprüche 2 bis 11, wobei

das organische Lösungsmittel oder das Gemisch von organischen Lösungsmitteln eine fluorierte organische Substanz oder ein Gemisch fluorierter organischer Substanzen ist.

**13.** Beschichtung nach einem der Ansprüche 1 und 3 bis 12 oder Verfahren nach einem der Ansprüche 2 bis 12, wobei das organische Lösungsmittel oder das Gemisch von organischen Lösungsmitteln eine Siedetemperatur zwischen 60˚C und 140˚C aufweist.

**14.** Beschichtung nach einem der Ansprüche 1 und 3 bis 13 oder Verfahren nach einem der Ansprüche 2 bis 13, wobei das organische Lösungsmittel Perfluor(2-butyltetrahydrofuran) oder ein chlorierter Fluorkohlenstoff ist.

**15.** Beschichtung nach einem der Ansprüche 1 und 3 bis 14 oder Verfahren nach einem der Ansprüche 2 bis 14, wobei das organische Lösungsmittel oder das Gemisch von organischen Lösungsmitteln ein Gemisch von 3,3-Dichlor-1,1,1,2,2-pentafluorpropan und 1,3-Dichlor-1,1,2,2,3-pentafluorpropan umfasst.

**16.** Beschichtung nach einem der Ansprüche 1 und 3 bis 15 oder Verfahren nach einem der Ansprüche 2 bis 15, wobei das organische Lösungsmittel oder das Gemisch von organischen Lösungsmitteln N,N-Dimethylacetamid, N,N-Dimethylformamid, Dimethylsulfoxid, Aceton, Cyclohexanon, Methylisobutylketon, Methylethylketon, N-Methylpyrrolidon, 1,4-Dioxan oder ein Gemisch davon ist.

**17.** Beschichtung nach einem der Ansprüche 2 bis 16, die ferner ein Polymer auf der Basis von pyrolytischem Kohlenstoff oder Poly(para-xylylen) umfasst.

**18.** Beschichtung nach einem der Ansprüche 1 und 3 bis 17 oder Verfahren nach einem der Ansprüche 2 bis 16, wobei die therapeutische Substanz Rapamycin, 40-O-(2-Hydroxy)ethylrapamycin, 40-O-[2-(2-Hydroxy)ethoxy]ethylrapamycin, 40-O-(3-Hydroxy)propylrapamycin oder 40-O-Tetrazolrapamycin ist.

**19.** Beschichtung nach einem der Ansprüche 1 und 3 bis 18, die ferner ein Blockcopolymer umfasst.

**20.** Beschichtung nach Anspruch 19, bei der das Blockcopolymer ein Polyharnstoff, ein Polyurethan, ein Polyharnstofffurethan, ein Styrol-Butadien-Styrol-Triblockcopolymer, ein Styrol-Isopren-Styrol-Triblockcopolymer oder ein Styrol-Ethylen/Propylen-Styrol-Triblockcopolymer ist.

**21.** Verfahren nach einem der Ansprüche 2 bis 16 und 18, das ferner das Einbeziehen eines Polymers auf der Basis von pyrolytischem Kohlenstoff oder von Poly(para-xylylen) in die Beschichtung umfasst.

**22.** Beschichtung nach einem der Ansprüche 1 und 3 bis 20 oder Verfahren nach einem der Ansprüche 2 bis 16, 18 und 21, wobei das erste Polymer ein Copolymer aus Ethylen und Vinylalkohol (EVAL) ist.

**23.** Implantierbare medizinische Vorrichtung, die eine Beschichtung gemäß der Definition in einem der Ansprüche 1, 3 bis 20 und 22 umfasst.

## Revendications

**1.** Revêtement pour dispositif médical implantable, lequel revêtement comprend :

a) une couche de médicament et polymère, laquelle couche de médicament et polymère comprend une substance thérapeutique et un premier polymère, lequel premier polymère est choisi parmi un copolymère d'éthylène et d'alcool vinylique (EVAL), un copolymère bloc polyurée, un copolymère tribloc poly(styrène-butadiène-styrène), un copolymère tribloc poly(styrène-isoprène-styrène), un polyimide, un polyéther, une résine époxy, de la rayonne, de la rayonne-triacétate, de l'acétate de cellulose, du butyrate de cellulose, de l'acéto-butyrate de cellulose, de la cellophane, du nitrate de cellulose, du propionate de cellulose, un éther de cellulose, de la carboxyméthyl-cellulose, un poly(acide aminé), un poly(cyano-acrylate), un poly(triméthylène carbonate), un poly(imino-carbonate), un copoly(éther-ester), un poly(alkylène oxalate), un polyphosphazène, de la fibrine, du fibrinogène, de la cellulose, de l'amidon, du collagène, de l'acide hyaluronique, un polyuréthane, une silicone, un polyester, un copolymère d'éthylène et d'alpha-oléfine et polyisobutylène, un polymère ou copolymère polyacrylique, un poly(ester de vinyle), du poly(chlorure de vinylidène), du polyacrylonitrile, une poly(vinyl-cétone), un poly(vinyl-arène), un polyamide, une résine alkyde, un polycarbonate, et un poly(oxyméthylène) ;

b) et une couche de dessus, laquelle couche de dessus comprend un deuxième polymère fluoré, lequel polymère fluoré est soluble dans un solvant organique ou dans un mélange de solvants organiques.

**2.** Procédé de production d'un dispositif médical implantable doté d'un revêtement, lequel revêtement comprend :

a) une couche de médicament et polymère, laquelle couche de médicament et polymère comprend une substance thérapeutique et un premier polymère, lequel premier polymère est choisi parmi un copolymère d'éthylène et d'alcool vinylique (EVAL), un copolymère bloc polyurée, un copolymère tribloc poly(styrène-butadiène-styrène), un copolymère tribloc poly(styrène-isoprène-styrène), un polyimide, un polyéther, une résine époxy, de la rayonne, de la rayonne-triacétate, de l'acétate de cellulose, du butyrate de cellulose, de l'acéto-butyrate de cellulose, de la cellophane, du nitrate de cellulose, du propionate de cellulose, un éther de cellulose, de la carboxyméthyl-cellulose, un poly(acide aminé), un poly(cyano-acrylate), un poly(triméthylène carbonate), un poly(imino-carbonate), un copoly(éther-ester), un poly(alkylène oxalate), un polyphosphazène, de la fibrine, du fibrinogène, de la cellulose, de l'amidon, du collagène, de l'acide hyaluronique, un polyuréthane, une silicone, un polyester, un copolymère d'éthylène et d'alpha-oléfine et polyisobutylène, un polymère ou copolymère polyacrylique, un poly(ester de vinyle), du poly(chlorure de vinylidène), du polyacrylonitrile, une poly(vinyl-cétone), un poly(vinyl-arène), un polyamide, une résine alkyde, un polycarbonate, et un poly(oxyméthylène) ;
b) et une couche de dessus, laquelle couche de dessus comprend un deuxième polymère fluoré, lequel polymère fluoré est soluble dans un solvant organique ou dans un mélange de solvants organiques ;
lequel procédé comporte les étapes suivantes :

i) former ladite couche de médicament et polymère en dissolvant ledit premier polymère et ladite substance thérapeutique dans un premier solvant et en appliquant la solution résultante sur un dispositif médical implantable ;
ii) et former ladite couche de dessus en appliquant sur le dispositif médical implantable une solution dudit deuxième polymère fluoré dans ledit solvant organique ou mélange de solvants organiques et en laissant s'évaporer le ou les solvant(s) organique(s).

**3.** Revêtement conforme à la revendication 1, ou procédé conforme à la revendication 2, dans lequel le dispositif médical est une prothèse endovasculaire (stent).

**4.** Revêtement conforme à la revendication 1 ou 3, ou procédé conforme à la revendication 2 ou 3, dans lequel le polymère fluoré est un polymère à base d'oléfine.

**5.** Revêtement conforme à la revendication 1, 3 ou 4, ou procédé conforme à l'une des revendications 2 à 4, dans lequel le polymère fluoré est du poly(fluorure de vinylidène), un copolymère de fluorure de vinylidène et d'hexafluoropropène, du poly(tétrafluoroéthylène), un copolymère d'éthylène et de propylène fluoré, du poly(hexafluoropropène), du poly-(chlorotrifluoroéthylène), un copolymère de fluorure de vinylidène et de tétrafluoroéthylène, un copolymère de tétrafluoroéthylène et d'hexafluoro-propène, un copolymère de tétrafluoroéthylène et d'alcool vinylique, un copolymère de tétrafluoroéthylène et d'acétate de vinyle, un copolymère de tétrafluoroéthylène et de propène, un copolymère d'hexafluoropropène et d'alcool vinylique, un copolymère de tétrafluoro-éthylène et de fluoro-méthyl-vinyl-éther, un copolymère d'éthylène et de tétrafluoroéthylène, un copolymère d'éthylène et d'hexafluoropropène, un copolymère de fluorure de vinylidène et de chlorotrifluoroéthylène, une silicone fluorée, ou un mélange de tels polymères.

**6.** Revêtement conforme à l'une des revendications 1 et 3 à 5, ou procédé conforme à l'une des revendications 2 à 5, dans lequel le polymère fluoré présente un paramètre de solubilité inférieur à 11 $cal^{1/2}/cm^3$

**7.** Revêtement conforme à l'une des revendications 1 et 3 à 6, ou procédé conforme à l'une des revendications 2 à 6, dans lequel le polymère fluoré comprend des motifs dérivés d'esters cycliques fluorés.

**8.** Revêtement ou procédé, conforme à la revendication 7, dans lequel le polymère fluoré comprend un copolymère de perhalogéno-2,2-diméthyl-1,3-dioxole et de perfluoro-2-méthylène-méthyl-1,3-dioxolane, un copolymère de perfluoro-oléfine et de perfluoro-2,2-diméthyl-1,3-dioxole, ou un copolymère de perfluoro-alkyl-vinyl-éther et de perfluoro-2,2-diméthyl-1,3-dioxole.

**9.** Revêtement ou procédé, conforme à la revendication 8, dans lequel le copolymère de perfluoro-oléfine et de perfluoro-2,2-diméthyl-1,3-dioxole est un copolymère de tétrafluoroéthylène et de perfluoro-2,2-diméthyl-1,3-dioxole.

**10.** Revêtement conforme à l'une des revendications 1 et 3 à 9, ou procédé conforme à l'une des revendications 2 à 9, dans lequel le polymère fluoré comprend des motifs dérivés d'éthers vinyliques fluorés.

**11.** Revêtement ou procédé, conforme à la revendication 10, dans lequel le polymère fluoré est un poly(perfluorobutényl-vinyl-éther).

**12.** Revêtement conforme à l'une des revendications 1 et 3 à 11, ou procédé conforme à l'une des revendications 2 à 11, dans lequel le solvant organique ou mélange de solvants organiques est une substance organique fluorée ou un mélange de substances organiques fluorées.

**13.** Revêtement conforme à l'une des revendications 1 et 3 à 12, ou procédé conforme à l'une des revendications 2 à 12, dans lequel le solvant organique ou mélange de solvants organiques présente une température d'ébullition située entre 60 ˚C et 140 ˚C.

**14.** Revêtement conforme à l'une des revendications 1 et 3 à 13, ou procédé conforme à l'une des revendications 2 à 13, dans lequel le solvant organique ou mélange de solvants organiques est du 2-butyl-tétrahydrofurane perfluoré ou un fluorocarbure chloré.

**15.** Revêtement conforme à l'une des revendications 1 et 3 à 14, ou procédé conforme à l'une des revendications 2 à 14, dans lequel le solvant organique ou mélange de solvants organiques comprend un mélange de 3,3-dichloro-1,1,1,2,2-pentafluoropropane et de 1,3-dichloro-1,1,2,2,3-pentafluoropropane.

**16.** Revêtement conforme à l'une des revendications 1 et 3 à 15, ou procédé conforme à l'une des revendications 2 à 15, dans lequel le solvant organique ou mélange de solvants organiques est du N,N-diméthyl-acétamide, du N,N-diméthyl-formamide, du diméthyl-sulfoxyde, de l'acétone, de la cyclohexanone, de la méthyl-isobutyl-cétone, de la méthyl-éthyl-cétone, de la N-méthyl-pyrrolidone ou du 1,4-dioxane, ou un mélange de ces solvants.

**17.** Revêtement conforme à l'une des revendications 2 à 16, qui comprend en outre un polymère pyrolytique à base de carbone ou un poly(para-xylylène).

**18.** Revêtement conforme à l'une des revendications 1 et 3 à 17, ou procédé conforme à l'une des revendications 2 à 16, dans lequel la substance thérapeutique est de la rapamycine, de la 40-O-(2-hydroxy-éthyl)-rapamycine, de la 40-O-[2-(2-hydroxy-éthoxy)éthyl]-rapamycine, de la 40-O-(3-hydroxy-propyl)-rapamycine, ou de la 40-O-tétrazolyl-rapamycine.

**19.** Revêtement conforme à l'une des revendications 1 et 3 à 18, qui comprend en outre un copolymère à blocs.

**20.** Revêtement conforme à la revendication 19, dans lequel le copolymère à blocs est une polyurée, un polyuréthane, un poly-urée-uréthane, un copolymère tribloc poly(styrène-butadiène-styrène), un copolymère tribloc poly(styrène-isoprène-styrène), ou un copolymère tribloc poly(styrène-éthylène/propylène-styrène).

**21.** Procédé conforme à l'une des revendications 2 à 16 et 18, qui comporte en outre le fait d'introduire dans le revêtement un polymère pyrolytique à base de carbone ou un poly(para-xylylène).

**22.** Revêtement conforme à l'une des revendications 1 et 3 à 20, ou procédé conforme à l'une des revendications 2 à 16, 18 et 21, dans lequel le premier polymère est un copolymère d'éthylène et d'alcool vinylique (EVAL).

**23.** Dispositif médical implantable, doté d'un revêtement conforme à l'une des revendications 1, 3 à 20 et 22.

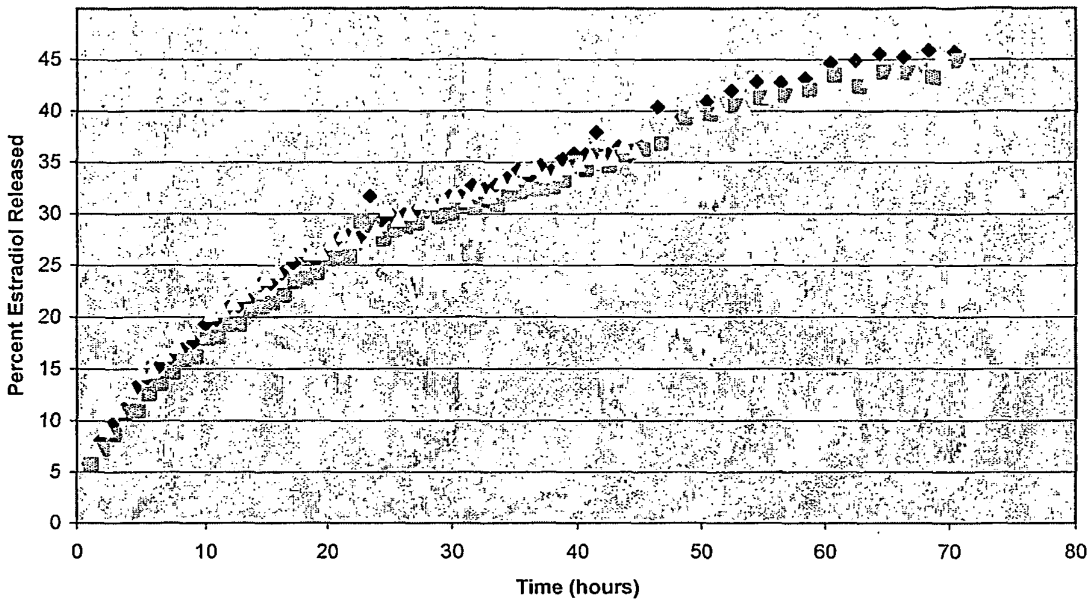
Percent Estradiol Released
0
5
10
15
20
25
30
35
40
45
0
10
20
30
40
50
60
70
80
Time (hours)

Fig. 1

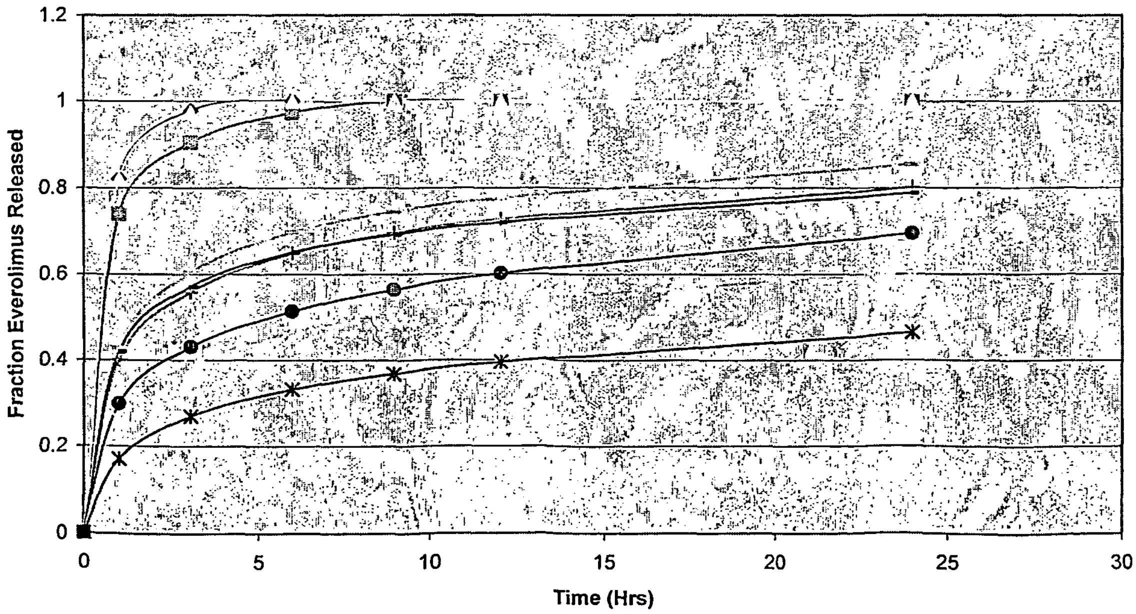
Fraction Everolimus Released
0
0.2
0.4
0.6
0.8
1
1.2
0
5
10
15
20
25
30
Time (Hrs)

Fig. 2

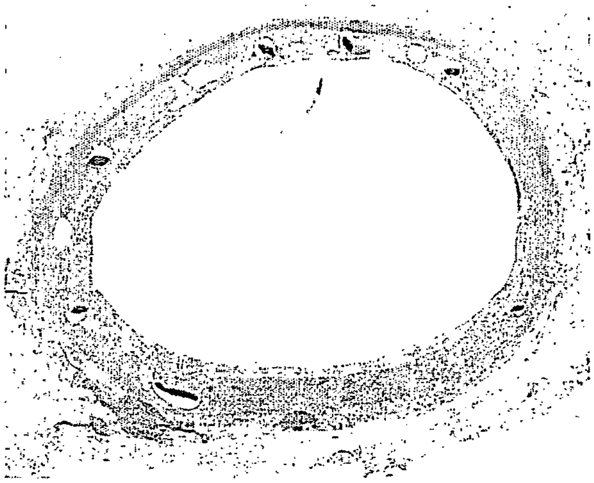

Fig. 3

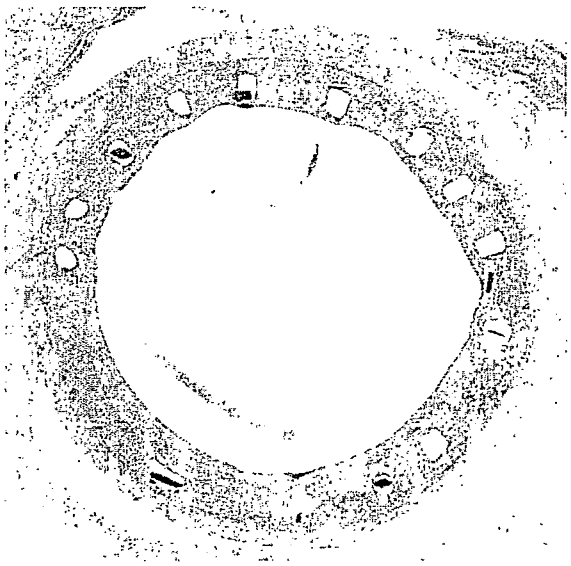

Fig. 4

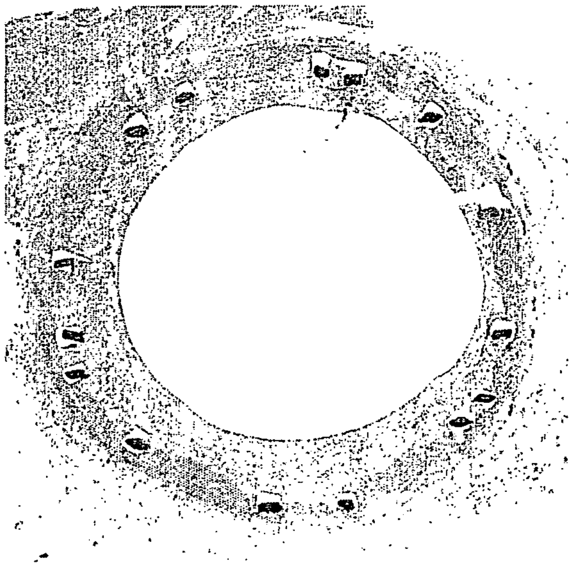

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0226271 A
- US 6153252 A
- US 5820917 A
- US 5837008 A
- US 20020122877 A
- US 5879697 A
- US 5928279 A
- US 5873904 A
- US 6299604 B
- WO 0130403 A
- WO 0187368 A

### Non-patent literature cited in the description

- **R.S. SCHWARTZ et al.** Restenosis and the Proportional Neointimal Response to Coronary Artery Injury: Results in a Porcine Model. *Journal of American College of Cardiology,* 1992, vol. 19, 267-274